# EUROPEAN PATENT APPLICATION

(11) **EP 4 350 005 A1**
(43) Date of publication of application: **10.04.2024**
(21) Application number: 22811689.3
(22) Date of filing: 27.05.2022
(51) Int. Cl.: C12Q 1/686, C12Q 1/6869, C40B 40/08

(54) **METHOD FOR SCREENING EXTERNALLY-INTRODUCED MRNA CAPABLE OF EXISTING FOR LONG TIME IN CELL**

(30) Priority: 28.05.2021 KR 20210069538
(71) Applicant: RNAGENE Inc., Seoul 05855 (KR)
(72) Inventor: LEE, Woo Ghil, Seoul 05834 (KR)
(74) Representative: Murgitroyd & Company
(86) International application number: PCT/KR2022/007577
(87) International publication number: WO 2022/250498

(57) **Abstract**

The present disclosure relates to a method of screening for and producing an mRNA sequence with improved intracellular stability that can exist in cells for a long period of time. According to a method according to an aspect, an mRNA sequence with improved intracellular stability can be screened for and produced by introducing a variation into a nucleotide sequence encoding a target protein such that there is no change in an amino acid sequence of the target protein. When the method is used, there is the advantage of improving the expression level of the target protein *in vivo.*

## Description

### Technical Field

The present disclosure relates to a method of screening for and producing an mRNA sequence with improved intracellular stability that can exist in cells for a long period of time.

### Background Art

mRNA has recently attracted great attention as a promising new drug, going beyond simply a material that delivers genetic information. Synthetic mRNA, which is structurally similar to natural RNA, is used to temporarily produce a target protein so that desired activity can be exhibited. Currently, mRNA-based cancer immunotherapeutic agents and infectious disease vaccines are in clinical trials.

The concept of nucleic acid-encoded drugs began when Wolff et al. showed that, when in vitro transcribed (IVT) mRNA or plasmid DNA (pDNA) were injected into skeletal muscle of mice, an encoded protein was expressed in the muscle. Conceptually, there are several important differences between IVT mRNA-based treatment methods and other nucleic acid-based treatments. IVT mRNA does not need to enter the nucleus to exert its function, and once mRNA is introduced into the cytoplasm in various ways, the mRNA is immediately translated. In contrast, DNA therapeutic agents must access the nucleus for transcription into RNA, and their function requires the breakdown of the nuclear membrane during cell division. In addition, unlike DNA and viral vectors, IVT mRNA-based therapeutics have the advantage of not being integrated into the genome, so there is no possibility of insertional mutagenesis.

Meanwhile, to use mRNA as a drug, it is first necessary to understand the relative stability control of mRNA, which is an important step in intracellular gene expression. The stability of mRNA plays an important role in determining not only the level of expression but also the production rate of a gene product. Generally, many short-lived proteins are encoded by short-lived mRNAs. mRNA encoding stable proteins, such as α-globin in blood cells, is known to have a considerably long half-life. In addition, cells use surveillance mechanisms to determine and shorten the half-life of mRNAs including nonsense codon mutations. Thus, these changes in mRNA stability can have profound consequences on cellular response and function.

Therefore, to improve the function of an mRNA therapeutic agent, there is a need to improve *in vivo* stability, and thus, in the present disclosure, a method for screening for and producing mRNA with improved intracellular stability was developed.

### Disclosure

### Technical Problem

An aspect provides a method of screening for an mRNA sequence with improved intracellular stability, the method including: (A) designing a template sequence including a sequence in which a variation has been introduced into a nucleotide sequence encoding a target protein such that there is no change in an amino acid sequence of the target protein; (B) producing primers on the basis of the designed template sequence; (C) constructing a library including various sequences into which variations have been introduced, using sequences of the produced primers; and (D) selecting an mRNA sequence with improved intracellular stability from the constructed library.

### Technical Solution

An aspect is to provide a method of screening for an mRNA sequence with improved intracellular stability, the method including: (A) designing a template sequence including a sequence in which a variation has been introduced into a nucleotide sequence encoding a target protein such that there is no change in an amino acid sequence of the target protein; (B) producing primers on the basis of the designed template sequence; (C) constructing a library including various sequences into which variations have been introduced, using sequences of the produced primers; and (D) selecting an mRNA sequence with improved intracellular stability from the constructed library.

The method relates to production of a sequence with improved intracellular stability among various nucleotide sequences encoding the target protein, and specifically, the method may include selecting a sequence with excellent intracellular stability from among various sequences in which variations have been introduced into wild-type nucleotide sequences encoding the target protein such that there is no change in the amino acid sequence of the target protein.

The sequence with improved intracellular stability may mean a sequence that has a reduced degree of degradation by ribonuclease (RNase) or the like and/or has an excellent expression level of the target protein *in vivo*/in cells. The sequence may be a DNA sequence or an RNA sequence, which is a nucleic acid sequence, and specifically, may be a cDNA sequence or mRNA sequence encoding the target protein.

The term "target protein" as used herein refers to a protein to be produced by those of ordinary skill in the art, and refers to cDNA or mRNA screened for by using the method of the present disclosure, or any protein that can be expressed by introducing the cDNA or the mRNA into a host cell.

The target protein or mRNA encoding the same may be used as a cancer immunotherapeutic agent, a cancer therapeutic vaccine, an infectious disease vaccine, a protein replacement therapeutic agent, an allergy therapeutic agent, or an immune disease therapeutic agent.

The term "coding sequence" as used herein refers to a double-stranded DNA or mRNA sequence that is transcribed and translated into a polypeptide in a cell *in vitro* or *in vivo* when placed under the control of suitable regulatory sequences, and can be used interchangeably with the term "nucleotide sequence encoding," "sequence encoding," or the like. The boundaries of the coding sequence are determined by an initiation codon at the 5' (amino) end and a stop codon at the 3' (carboxyl) end. The coding sequence may also include prokaryotic sequences, cDNA from eukaryotic mRNA, genomic DNA sequences from eukaryotic (e.g., mammalian) DNA, and synthetic DNA sequences.

The nucleotide sequence encoding the target protein may be a cDNA sequence consisting of only exons required for translation of mRNA.

In step (A), the introduction of the variation may include introducing a redundancy codon encoding the same amino acid into all or part of the nucleotide sequence encoding the target protein, and specifically, may include introducing N (A, G, C, or T), R (A or G), Y (C or T), or H (A, C, or T) into the third nucleotide of each codon. Thus, any base that is not specifically limited to the third nucleotide of each codon may be introduced, and numerous nucleotide sequences are created depending on the combination of introduced nucleotides, through which a library of nucleotide sequences encoding the same amino acid sequence may be constructed.

The term "codon" as used herein refers to the triplet code of mRNA, in which three nucleotide sequences of mRNA are combined to form one amino acid, and in this process, the combination of three nucleotides is interpreted as integrated coding information, which is therefore called a triplet code, and is expressed as a unit called a codon.

The term "redundancy codon" as used herein refers to a set of codons encoding the same amino acid, and may be used interchangeably with the term "synonymous codon." There are a total of 64 types of codons, and 61 types, excluding 3 types of stop codons, encode 20 types of amino acids. There are as few as 1 and as many as 6 types of codons encoding one amino acid. Specifically, the former includes methionine and tryptophan, and the latter includes leucine, arginine, and serine. There are 2, 3, and 4 codons that encode the remaining 15 types of amino acids. This fact means that there are numerous types of mRNA that can encode one protein.

In step (A), to impart diversity to the nucleotide sequence encoding the target protein such that there is no change in the amino acid sequence of the target protein, a codon encoding each amino acid may be modified as follows: uuY for phenylalanine; uuR or cuN for leucine; auH for isoleucine; guN for valine; ucN and agY for serine; ccN for proline; acN for threonine; gcN for alanine; uaY for tyrosine; caY for histidine; caR for glutamine; aaY for asparagine; aaR for lysine; gaY for aspartic acid; gaR for glutamic acid; ugY for cysteine; cgN or agR for arginine; and ggN for glycine. In addition, mutations may not be introduced into tryptophan, methionine, which is an initiation codon, and a stop codon.

The template sequence designed in step (A) may further include at least one selected from the group consisting of a promoter, an untranslated region (UTR), a 5' cap, and a 3' polyadenylic acid sequence (poly-A), and in addition thereto, may further include components that regulate the expression of the target protein.

The term "promoter" as used herein refers to a part of DNA that is involved in the binding of RNA polymerase to initiate transcription. The promoter is generally located adjacent to and upstream of a target gene, is a site to which RNA polymerase or a transcription factor, which is a protein that induces RNA polymerase, binds, and may guide the enzyme or protein to be positioned at the correct transcription initiation site. That is, the promoter has a specific gene sequence which is located at the 5' region of a gene to be transcribed in a sense strand and induces the initiation of mRNA synthesis for a target gene when an RNA polymerase binds to a corresponding site directly or via a transcription factor.

The promoter may be constitutive or inducible as a general promoter, and may include, but is not limited to: lac, tac, T3, and T7 promoters for prokaryotic cells; and for eukaryotic cells, a monkey virus 40 (SV40) promoter, a mouse mammary tumor virus (MMTV) promoter, a long terminal repeat (LTR) promoter of human immunodeficiency virus (HIV), molonivirus, cytomegalovirus (CMV), Epstein barr virus (EBV), rous sarcoma virus (RSV) promoters, a β-actin promoter, and promoters derived from human hemoglobin, human muscle creatine, and human metallothionein.

The term "untranslated region (UTR)" as used herein is also referred to as an untranslated region, and refers to a portion of an mRNA chain that does not serve as a template for a protein gene, that is, a portion that is not translated. Generally, the UTR includes 5'-UTR and 3'-UTR.

The term "5' cap" or "end cap" as used herein refers to a 7-methyl guanosine (7mG) cap and an anti-reverse cap analog (ARCA) that are chemically bonded to the terminal 5' nucleotide of an RNA molecule.

The term "poly-adenylic acid sequence (poly-A)" or "poly-A tail" as used herein refers to a series of contiguous adenylic acids (polyadenylates) added after transcription of an RNA molecule such as mRNA at the 3' end.

The promoter, the untranslated region (UTR), the 5' cap, and/or the 3' polyadenylic acid sequence (poly-A) included in the template may be operably linked to the sequence encoding the target protein.

The term "operably linked" as used herein refers to a state in which a nucleic acid expression control sequence and a nucleic acid sequence encoding a target protein or peptide are functionally linked to perform a general function. For example, the promoter and the nucleic acid sequence encoding a protein or peptide may be operably linked to affect the expression of the coding sequence. Operational linkage with an expression vector may be formed by genetic recombination technology well known in the art, and site-specific DNA cleavage and linkage may be achieved by using enzymes and the like generally known in the art.

The 5' untranslated region, the promoter, the sequence encoding the target protein, the 3' untranslated region, and the polyadenylic acid sequence may be linked in the stated order to constitute a template, and may be directly linked to each other or may be indirectly linked to each other via a linker.

The linker is not particularly limited thereto as long as the linker does not affect the expression of the target protein, and may consist of, for example, 3 to 21 nucleic acids.

Step (A) may further include setting the designed template sequence as one or more regions, and specifically, may further include setting the designed template sequence as two or more divided regions. The template sequence designed in step (A) may be divided into two or more regions, and specifically, the template sequence may be set as two or more regions by dividing each region to a size of 10 bp to 1,000 bp, and the divided regions may be named from a first region to an n^{th} region (n is an integer of 2 or more), from the 5' end. In addition, the divided regions may have the same size or different sizes.

The size of the divided regions may range from 10 bp to 1,000 bp, and may be in a range of, for example, 10 bp to 1,000 bp, 10 bp to 900 bp, 10 bp to 800 bp, 10 bp to 700 bp, 10 bp to 600 bp, 10 bp to 500 bp, 10 bp to 400 bp, 10 bp to 300 bp, 10 bp to 200 bp, 10 bp to 180 bp, 10 bp to 150 bp, 10 bp to 140 bp, 10 bp to 130 bp, 10 bp to 120 bp, 10 bp to 110 bp, 10 bp to 100 bp, 30 bp to 1,000 bp, 30 bp to 900 bp, 30 bp to 800 bp, 30 bp to 700 bp, 30 bp to 600 bp, 30 bp to 500 bp, 30 bp to 400 bp, 30 bp to 300 bp, 30 bp to 200 bp, 30 bp to 180 bp, 30 bp to 150 bp, 30 bp to 140 bp, 30 bp to 130 bp, 30 bp to 120 bp, 30 bp to 110 bp, 30 bp to 100 bp, 50 bp to 1,000 bp, 50 bp to 900 bp, 50 bp to 800 bp, 50 bp to 700 bp, 50 bp to 600 bp, 50 bp to 500 bp, 50 bp to 400 bp, 50 bp to 300 bp, 50 bp to 200 bp, 50 bp to 180 bp, 50 bp to 150 bp, 50 bp to 140 bp, 50 bp to 130 bp, 50 bp to 120 bp, 50 bp to 110 bp, 50 bp to 100 bp, 60 bp to 1,000 bp, 60 bp to 900 bp, 60 bp to 800 bp, 60 bp to 700 bp, 60 bp to 600 bp, 60 bp to 500 bp, 60 bp to 400 bp, 60 bp to 300 bp, 60 bp to 200 bp, 60 bp to 180 bp, 60 bp to 150 bp, 60 bp to 140 bp, 60 bp to 130 bp, 60 bp to 120 bp, 60 bp to 110 bp, 60 bp to 100 bp, 70 bp to 1,000 bp, 70 bp to 900 bp, 70 bp to 800 bp, 70 bp to 700 bp, 70 bp to 600 bp, 70 bp to 500 bp, 70 bp to 400 bp, 70 bp to 300 bp, 70 bp to 200 bp, 70 bp to 180 bp, 70 bp to 150 bp, 70 bp to 140 bp, 70 bp to 130 bp, 70 bp to 120 bp, 70 bp to 110 bp, 70 bp to 100 bp, 80 bp to 1,000 bp, 80 bp to 900 bp, 80 bp to 800 bp, 80 bp to 700 bp, 80 bp to 600 bp, 80 bp to 500 bp, 80 bp to 400 bp, 80 bp to 300 bp, 80 bp to 200 bp, 80 bp to 180 bp, 80 bp to 150 bp, 80 bp to 140 bp, 80 bp to 130 bp, 80 bp to 120 bp, 80 bp to 110 bp, 80 bp to 100 bp, 90 bp to 1,000 bp, 90 bp to 900 bp, 90 bp to 800 bp, 90 bp to 700 bp, 90 bp to 600 bp, 90 bp to 500 bp, 90 bp to 400 bp, 90 bp to 300 bp, 90 bp to 200 bp, 90 bp to 180 bp, 90 bp to 150 bp, 90 bp to 140 bp, 90 bp to 130 bp, 90 bp to 120 bp, 90 bp to 110 bp, or 90 bp to 100 bp.

In addition, the ends of each of the divided regions may overlap each other, and accordingly, the ends of each divided region may overlap with the ends of the regions on both sides. The ends of each of the divided regions may be overlapping regions of 1 bp to 100 bp and for example, the 5' end of a second region may overlap with the 3' end of a first region, and the 3' end of the second region may overlap with the 5' end of a third region.

The size of the overlapping regions may range from 1 bp to 100 bp and may be in a range of, for example, 1 bp to 100 bp, 1 bp to 99 bp, 1 bp to 90 bp, 1 bp to 81 bp, 1 bp to 72 bp, 1 bp to 63 bp, 1 bp to 54 bp, 1 bp to 45 bp, 1 bp to 36 bp, 1 bp to 30 bp, 1 bp to 27 bp, 1 bp to 24 bp, 1 bp to 21 bp, 1 bp to 18 bp, 1 bp to 15 bp, 1 bp to 12 bp, 1 bp to 9 bp, 3 bp to 100 bp, 3 bp to 99 bp, 3 bp to 90 bp, 3 bp to 81 bp, 3 bp to 72 bp, 3 bp to 63 bp, 3 bp to 54 bp, 3 bp to 45 bp, 3 bp to 36 bp, 3 bp to 30 bp, 3 bp to 27 bp, 3 bp to 24 bp, 3 bp to 21 bp, 3 bp to 18 bp, 3 bp to 15 bp, 3 bp to 12 bp, 3 bp to 9 bp, 6 bp to 100 bp, 6 bp to 99 bp, 6 bp to 90 bp, 6 bp to 81 bp, 6 bp to 72 bp, 6 bp to 63 bp, 6 bp to 54 bp, 6 bp to 45 bp, 6 bp to 36 bp, 6 bp to 30 bp, 6 bp to 27 bp, 6 bp to 24 bp, 6 bp to 21 bp, 6 bp to 18 bp, 6 bp to 15 bp, 6 bp to 12 bp, 6 bp to 9 bp, 9 bp to 100 bp, 9 bp to 99 bp, 9 bp to 90 bp, 9 bp to 81 bp, 9 bp to 72 bp, 9 bp to 63 bp, 9 bp to 54 bp, 9 bp to 45 bp, 9 bp to 36 bp, 9 bp to 30 bp, 9 bp to 27 bp, 9 bp to 24 bp, 9 bp to 21 bp, 9 bp to 18 bp, 9 bp to 15 bp, 9 bp to 12 bp, 12 bp to 100 bp, 12 bp to 99 bp, 12 bp to 90 bp, 12 bp to 81 bp, 12 bp to 72 bp, 12 bp to 63 bp, 12 bp to 54 bp, 12 bp to 45 bp, 12 bp to 36 bp, 12 bp to 30 bp, 12 bp to 27 bp, 12 bp to 24 bp, 12 bp to 21 bp, 12 bp to 18 bp, 12 bp to 15 bp, 15 bp to 100 bp, 15 bp to 99 bp, 15 bp to 90 bp, 15 bp to 81 bp, 15 bp to 72 bp, 15 bp to 63 bp, 15 bp to 54 bp, 15 bp to 45 bp, 15 bp to 36 bp, 15 bp to 30 bp, 15 bp to 27 bp, 15 bp to 24 bp, 15 bp to 21 bp, or 15 bp to 18 bp.

In addition, variations may not be introduced into the overlapping portions of each of the divided regions. This is to induce complementary binding between a primer-primer or primer-PCR product when PCR is subsequently performed by using primers synthesized using the divided regions.

Step (B) is a step of designing and producing primer sequences for constructing a library including various sequences encoding the target protein on the basis of the template sequence into which a variation has been introduced, and may include synthesizing primers on the basis of sequences of the divided regions of the template.

Specifically, in step (B), primers are synthesized on the basis of the sequences of the divided regions of the template or sequences complementary thereto, and the primers may be synthesized so as to include the sequences of the divided regions of the template or sequences complementary thereto. Specifically, the sequences of the divided regions of the template or sequences complementary thereto may constitute primer sequences. The synthesized primers may be named from a first primer to an n^{th} primer (n is an integer of 2 or more).

In addition, the primer synthesis step may include synthesizing the complementary sequences as primers in the remaining regions (2^{nd} region to n^{th} region) excluding the first region, from the 5' end, and specifically, the remaining primers excluding the first primer, among the synthesized primers, may be synthesized on the basis of the complementary sequences of the divided regions.

Step (C) is a step of constructing a library including various sequences encoding the target protein, including various variations by using the primer sequences produced in step (B), and specifically, may include performing PCR sequentially by using the synthesized primers and/or PCR products. For the sequential PCR, in the case of a template divided into n regions, PCR may be sequentially performed n-1 times. The sequential PCR reactions can be named from a first PCR to a (n-1)^{th} PCR.

For example, the first PCR of the sequential PCR is PCR performed by using a first primer and a second primer without a template, in which the 3' ends (overlapping portions of the template) of the first primer and the second primer complementarily bind and the first and second primers themselves act as a template. Next, the second PCR is PCR performed by using the first primer and a third primer and using a product of the first PCR as a template, in which the product of the first PCR and the 3' end (an overlapping portion of the template) of the third primer complementarily bind to each other.

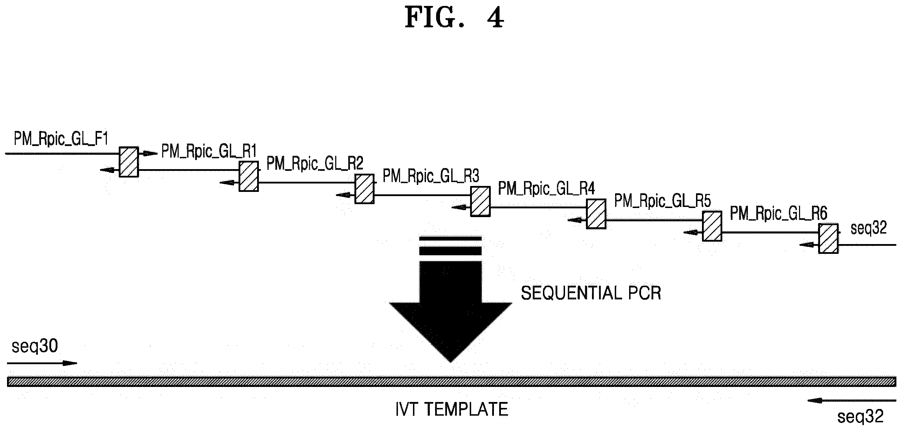

The sequential PCR is performed in the same manner as above, and finally, the first PCR is performed by using the first primer and the second primer (the 3' end of the first primer and the 3' end of the second primer correspond to the overlapping portions of the template, and complementarily bind to each other), and subsequently, the n-1^{th} PCR is performed by using the first primer and the n^{th} primer and using a product of the n-2^{th} PCR as a template (the 3' end of the product of the n-2^{th} PCR and the 3' end of the n^{th} primer correspond to the overlapping portions of the template and complementarily bind to each other) (n is an integer of 3 or more), and the final product of the PCR may be constructed as a library including various sequences on the basis of the template designed in step (A) (FIG. 4). Meanwhile, when the designed template is divided into two regions, a library may be constructed only through the first PCR performed by using the first primer and the second primer.

The library may include a DNA sequence or RNA sequence encoding the target protein, and the sequence encoding the target protein may further include at least one selected from the group consisting of a promoter, an untranslated region (UTR), a 5' cap, and a 3' polyadenylic acid sequence (poly-A).

Step (D) is a process of selecting an mRNA sequence with improved intracellular stability encoding the target protein from the constructed library, and may include the steps of: D-1) transfecting cells with the DNA sequence encoding the target protein included in the library or an RNA sequence produced therefrom; D-2) obtaining an RNA sequence with improved intracellular stability encoding the target protein from the transfected cells; and D-3) constructing a library by synthesizing DNA encoding the target protein from the obtained RNA sequence.

The DNA sequences included in the library are a collection of numerous template sequences including various cDNA sequences encoding the target protein such that there is no change in the amino acid sequence of the target protein.

The sequence used for transfection may be a DNA sequence included in the library or an RNA sequence produced therefrom. The RNA sequence is that transcribed from a sequence included in the library, and specifically, may be obtained through an *in vitro* transcription reaction.

The term "transfection" as used herein refers to introducing, into a host cell, the library sequence, an expression vector including the sequence, or an RNA sequence transcribed from the sequence for the purpose of the present disclosure, and enabling a nucleic acid molecule of the library sequence to be expressed in the host cell, and may be used interchangeably with the term "transduction" or "transformation." In addition, the nucleic acid molecule may include DNA and RNA in the form of a template sequence including a sequence encoding the target protein. The library sequence may be introduced in any form as long as the library sequence can be introduced into a host cell and expressed therein.

A suitable host cell for introduction into the library sequence may be a prokaryotic cell such as Escherichia coli, Bacillus subtilis, Streptomyces sp., Pseudomonas sp., Proteus mirabilis orStaphylococcus sp. In addition, the suitable host cell may be a fungus such as Aspergillus sp.,a yeast such as Pichia pastoris, Saccharomyces cerevisiae, Schizosaccharomyces sp., or Neurospora crassa, another lower eukaryotic cell, or a cell of a higher eukaryote, such as a cell of a plant or an insect. In addition, the suitable host cell may be a mammalian cell and specifically, may be, but is not limited to, a monkey kidney cell 7 (COS7) cell, an NSO cell, SP2/0, a Chinese hamster ovary (CHO) cell, W138, a baby hamster kidney (BHK) cell, MDCK, a myeloma cell line, a HeLa cell, a HuT 78 cell, or a HEK293 cell.

The transfection method includes any method of introducing a nucleic acid into an organism, a cell, a tissue or an organ, and may be performed by selecting a suitable standard technique according to the host cell known in the art, and examples of the suitable standard technique may include electroporation, protoplast fusion, calcium phosphate (CaPO₄) precipitation, calcium chloride (CaCl₂) precipitation, microinjection, stirring using silicon carbide fiber, agrobacteria-mediated transformation, a polyethylene glycol (PEG) method, a DEAE-dextran method, a cationic liposome method, a lithium acetate-DMSO method, lipofectamine, drying/inhibition mediated transformation method, and the like.

The term "expression vector" as used herein refers to a recombinant vector that can be introduced into a suitable host cell to express a target protein, and refers to a genetic construct including essential regulatory elements operably linked to express a gene insert. The term "operably linked" as used herein means that a nucleic acid expression control sequence and a nucleic acid sequence encoding a target protein are functionally linked to perform a general function. Operational linkage with a recombinant vector may be formed by genetic recombination technology well known in the art, and site-specific DNA cleavage and linkage may be easily achieved by using enzymes and the like generally known in the art.

A suitable expression vector of the present disclosure may include signal sequences for membrane targeting or secretion, in addition to expression control elements such as a promoter, an initiation codon, a stop codon, a polyadenylation signal, and an enhancer. The initiation codon and the stop codon are generally considered to be part of the nucleotide sequence encoding the target protein, and must be functional in a subject when a genetic construct is administered and must be in frame with the coding sequence.

In addition, the expression vector may include a selectable marker for selecting a host cell containing a vector. The selectable marker is used to select cells transformed with a vector, and markers that impart selectable phenotypes such as drug resistance, auxotrophy, resistance to cytotoxic agents, or expression of surface proteins may be used. In an environment treated with a selective agent, only cells expressing the selectable marker survive, so transformed cells can be selected. In addition, when the vector is a replicable expression vector, the vector may include a replication origin, which is a specific nucleic acid sequence where replication is initiated.

As recombinant expression vectors for inserting foreign genes, various forms of vectors such as plasmids, viruses, cosmids, and the like may be used. The type of recombinant vector is not particularly limited as long as the recombinant vector functions to express a desired gene and produce a desired protein in various types of host cells such as prokaryotic cells and eukaryotic cells. Specifically, the recombinant vector may be a vector capable of mass-producing a foreign protein having a similar form to the wild type while retaining a promoter having strong activity and a strong expression ability.

To express the target protein in the present disclosure, various combinations of hosts and vectors may be used. Expression vectors suitable for eukaryotic hosts may include, but are not limited to, expression control sequences derived from SV40, bovine papillomavirus, adenovirus, adeno-associated virus, cytomegalovirus, and retrovirus. Expression vectors that may be used in bacterial hosts may include, but are not limited to, bacterial plasmids obtained from *Escherichia coli,* including pET, pRSET, pBluescript, pGEX2T, a pUC vector, a T vector system, col El, pCR1, pBR322, pMB9, or a derivative thereof, plasmids having a broad host range such as RP4, phage DNA, e.g., a phage lambda derivative such as λgt10, λgt11, NM989, or the like, and other DNA phages such as M13 and filamentous single-stranded DNA phage. A 2°C plasmid, a derivative thereof, or the like may be used for yeast cells, and pVL941 or the like may be used for insect cells.

Step D-1) may further include a step of confirming an expression level of the target protein in the transfected cells, and the step may be to measure whether or not the target protein or a sequence encoding the target protein is expressed or to measure the expression level thereof. In addition, the step may be to confirm whether cells have been properly transfected.

A method for measuring the expression level of the protein may be western blotting, enzyme linked immunosorbent assay (ELISA), radioimmunoassay (RIA), radical immunodiffusion, Ouchterlony immunodiffusion, rocket immunoeletrophoresis, immunohistochemical staining, immunoprecipitation assay, complement fixation assay, immunofluorescence, immunochromatography, fluorescence-activated cell sorter (FACS) analysis, or protein chip technology.

A method of measuring the expression level of the sequence encoding the target protein, specifically mRNA or a gene, may be reverse transcriptase polymerase reaction (RT-PCR), competitive RT-PCR, real-time quantitative RT-PCR, quantitative RT-PCR, an RNase protection method, northern blotting, or DNA chip technology.

A step of evaluating the expression level of the target protein may involve using an agent for measuring the expression level. The term "agent for measuring the expression level" as used herein refers to a molecule that can be used to confirm the expression level of a specific protein or a gene encoding the same, and specifically, may include, but is not limited to, agents for detecting and/or amplifying the target protein or a gene encoding the same.

The term "agent for detecting a specific protein or a gene encoding the same" as used herein refers to an agent capable of specifically binding to and recognizing the specific gene or protein, or amplifying the same through detection. The term "agent for amplifying a specific gene or protein" as used herein refers to an agent capable of increasing the number of the specific genes or proteins by repeating replication, and may refer to a primer capable of specifically amplifying a polynucleotide including the gene or a probe capable of specifically binding thereto, but the present disclosure is not limited thereto.

To effectively evaluate the expression level of the target protein in the above step, a composition including a target protein or a gene encoding the same, or a primer, probe, nucleotide, antibody or antigen-binding fragment thereof, ligand, receptor, protein or combination thereof that specifically binds to mRNA may be used.

The term "primer" as used herein refers to a nucleic acid sequence having a free 3' hydroxyl group, which can form base pairs with a template complementary to a specific nucleotide sequence and serves as a starting point for replicating a template strand. A primer can initiate DNA synthesis in the presence of reagents for polymerization (e.g., DNA polymerase or reverse transcriptase) and four different nucleoside triphosphate in a suitable buffer and at an appropriate temperature. PCR conditions and the lengths of sense and antisense primers may be appropriately selected according to techniques known in the art.

The term "probe" as used herein refers to a nucleic acid fragment such as an RNA or DNA fragment that can specifically bind to a target nucleic acid, for example, mRNA, and the probe may be labeled so that the presence of specific mRNA and the amount and expression level thereof can be determined. The probe may be prepared in the form of an oligonucleotide probe, a single strand DNA probe, a double strand DNA probe, an RNA probe, or the like. Selection of a suitable probe and conditions for hybridization may be appropriately selected according to techniques known in the art. The probe may have a length of 10 nts to 100 nts, 15 nts to 100 nts, 10 nts to 80 nts, 10 nts to 50 nts, 10 nts to 30 nts, 10 nts to 20 nts, 15 nts to 80 nts, 15 nts to 50 nts, 15 nts to 30 nts, 15 nts to 20 nts, 20 nts to 100 nts, 20 nts to 80 nts, 20 nts to 50 nts, or 20 nts to 30 nts.

The primer or probe may be chemically synthesized by using a solid-phase support synthesis method using phosphoramidite or other synthesis methods widely known in the art. In addition, the nucleic acid sequence may be modified by using various methods known in the art. Examples of such modification may include methylation, capping, substitution of at least one natural nucleotide with an analog thereof, and modifications between nucleotides, such as modifications with a non-electrically charged linker (e.g., methyl phosphonate, phosphotriester, phosphoramidate, or carbamate) or an electrically charged linker (e.g., phosphorothioate or phosphorodithioate). In addition, the primer or probe may be modified using a marker capable of directly or indirectly providing a detectable signal. Examples of the marker may include a radioactive isotope, a fluorescent molecule, and biotin.

The term "antibody" as used herein is a term known in the art and refers to a specific immunoglobulin which is directed toward an antigenic site. The antibody can specifically bind to a target protein or a fragment thereof, and the fragment may be an immunogenic fragment and refers to a protein fragment having at least one epitope that can be recognized by an antibody against the protein.

Step D-1) may further include, after the transfection, a step of culturing the transfected cells, and specifically, may include a step of culturing cells into which a sequence encoding the target protein has been introduced, to select a sequence with improved intracellular or *in vivo* stability.

The culturing step may be carried out for, for example, 12 hours to 96 hours, 12 hours to 84 hours, 12 hours to 72 hours, 12 hours to 60 hours, 12 hours to 48 hours, 12 hours to 36 hours, 12 hours to 24 hours, 24 hours to 96 hours, 24 hours to 84 hours, 24 hours to 72 hours, 24 hours to 60 hours, 24 hours to 48 hours, 24 hours to 36 hours, 36 hours to 96 hours, 36 hours to 84 hours, 36 hours to 72 hours, 36 hours to 60 hours, 36 hours to 48 hours, 48 hours to 96 hours, 48 hours to 84 hours, 48 hours to 72 hours, 48 hours to 60 hours, 60 hours to 96 hours, 60 hours to 84 hours, 60 hours to 72 hours, 72 hours to 96 hours, or 72 hours to 84 hours.

Among various RNA sequences introduced into cells or RNA sequences translated from the DNA sequence during cell culture after the transfection, sequences with low *in vivo* stability are degraded inside the cells and cannot exist or have reduced expression levels, and only sequences with high intracellular stability are present or have improved expression levels. Thus, when RNA is obtained from the transfected cells in the next step D-2), RNA sequences which have improved intracellular or *in vivo* stability and are not degraded in cells, and improved expression levels compared to wild-type sequences into which no variation has been introduced may be obtained. In addition, by synthesizing DNA sequences from the obtained RNA sequences, a library (secondary library) consisting of selected sequences with excellent intracellular stability may be constructed.

In step (D), steps D-1) to D-3) may be repeatedly performed one or more times. Specifically, a DNA sequence included in the constructed secondary library or an RNA sequence produced therefrom may be transfected again into cells, and a more purified library (tertiary library) may be produced from the transfected cells. By repeating steps D-1) to D-3) one or more times, a library of sequences having superior intracellular stability may be obtained, thus providing a beneficial effect in screening for and producing an mRNA sequence with improved intracellular stability.

Step (D) may further include the steps of: D-4) cloning the DNA sequence encoding the target protein included in the library into a vector; D-5) transfecting cells with the vector or RNA produced therefrom; and D-6) obtaining and selecting RNA encoding the target protein from the transfected cells.

Specifically, since the library obtained through step D-3) includes various types of nucleotide sequences encoding the same amino acid sequence, step (D) may include a step of cloning the DNA sequence included in the library into a vector, to separate each nucleotide sequence and transfect cells with each nucleotide sequence. Cloned recombinant vectors include only one type of sequence per vector, and thus, cells transfected with the vector or RNA produced therefrom can express only one type of sequence.

Thus, the transfected cells (clone) in step D-6) include one type of coding sequence (DNA or RNA) per cell, and thus, RNA obtained from the cells (clone) may be one type of sequence obtained per cell (clone).

The transfected cells in step D-6) may be selected cells, and specifically, may be selected cells in which the expression level of the target protein or a sequence encoding the same is excellent. The selection process may include selecting by comparing the expression level of a control with the expression level of the target protein of the transfected cells, and the control may be a wild-type gene sequence into which no variation has been introduced.

Step (D) may further include D-7) a step of determining a sequence of the obtained RNA and DNA synthesized therefrom.

The determination of the sequence is to confirm sequence information by analyzing the nucleotide sequence of a target sequence, and may be performed by using a method known in the art, for example, Sanger Sequencing, Maxam-Gilbert Sequencing, or Next Generation Sequencing.

### Advantageous Effects

According to a method according to an aspect, an mRNA sequence with improved intracellular stability can be screened for and produced by introducing a variation into a nucleotide sequence encoding a target protein such that there is no change in an amino acid sequence of the target protein. When the method is used, there is the advantage of improving the expression level of the target protein *in vivo.*

### Description of Drawings

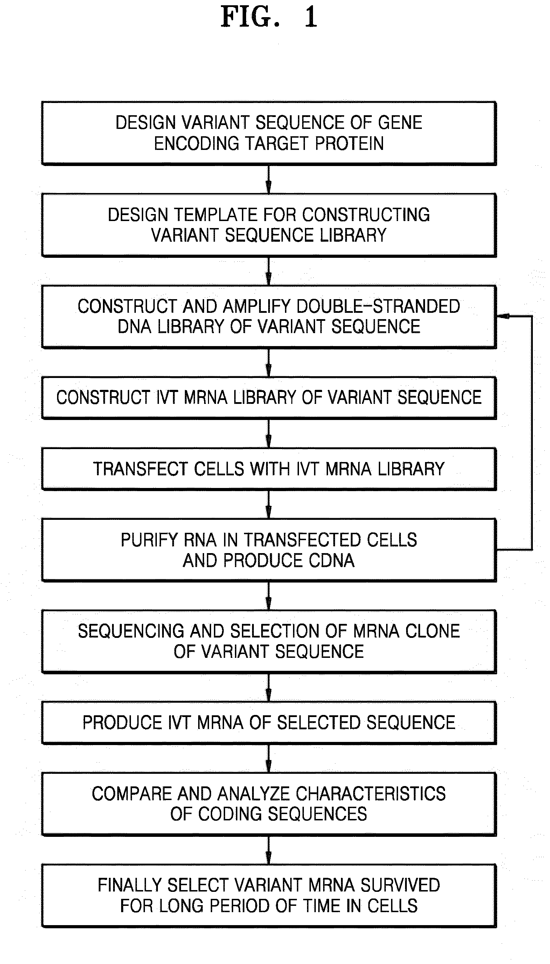

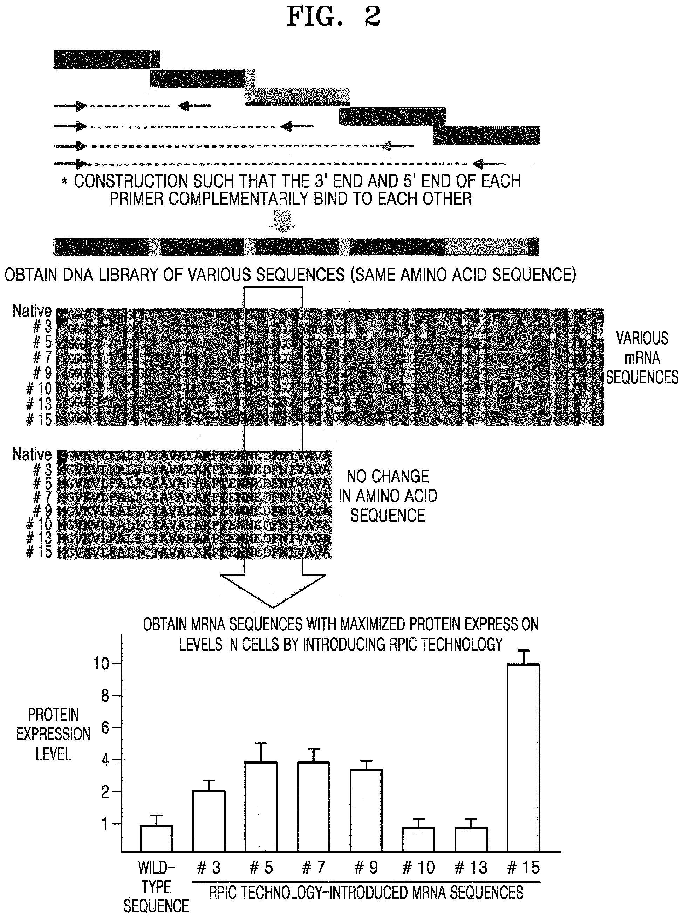

FIGS. 1 and 2 are a flowchart and a schematic view that illustrate processes of a method of screening for or producing an mRNA sequence with improved intracellular stability.
FIG. 3 is a schematic view illustrating a process of introducing a variation into a target protein (e.g., Gaussian luciferase) and a primer design process.
FIG. 4 is a schematic view illustrating a process of synthesizing a variation-introduced template through sequential PCR.
FIG. 5 is a view illustrating a process of performing sequential PCR by using designed/produced primers.
FIG. 6 is a view for comparing the expression level of a target protein between candidate mRNA sequences selected through the method of the present disclosure.
FIG. 7 is a view for comparing a secondary structure between a control and mRNA sequences with excellent intracellular stability selected through the method of the present disclosure.
FIG. 8 is a view for comparing the expression levels between sequences with excellent intracellular stability encoding a COVID19 antigen protein, selected using HeLa cells through the method of the present disclosure.
FIG. 9 is a diagram for comparing the expression levels between sequences with excellent intracellular stability encoding a COVID19 antigen protein, selected using HEK293T cells through the method of the present disclosure.
FIG. 10 is a view for comparing the expression levels between sequences with excellent intracellular stability encoding a recombinant protein including a COVID19 antigen protein, selected using HeLa cells through the method of the present disclosure.

### Best Mode

### Mode for Invention

Hereinafter, the present disclosure will be described in further detail with reference to the following examples. However, these examples are provided for illustrative purposes only and are not intended to limit the scope of the present disclosure.

### Example 1: Template design and library construction to maximize mRNA codon mutation

To explain the method of screening for and producing mRNA with improved stability, according to the present disclosure, the coding sequence of the Gaussian luciferase (GL) gene, which is a well-known secretory luciferase enzyme, was used as a representative example. The coding sequence was referred to the coding sequence of the Gaussia princeps luciferase gene in pCMV-GLuc_2, which is a cloning vector available from New England Biolabs, USA. A T7 promoter was located at the 5'-end region of the gene sequence to enable *in vitro* transcription (lVT), and UTR coding sequences known to increase mRNA stability were respectively inserted into the 5' end and 3' end regions of the gene sequence. In addition, a polyadenylation sequence was located at the 3' end region and designed to be used as mRNA immediately after IVT. A template (wild-type Gaussian luciferase (GL) gene template) sequence including the wild-type Gaussian luciferase gene sequence with no introduced variation was represented by a sequence of SEQ ID NO: 1.

Next, while maintaining each amino acid sequence of a protein translated from the open reading frame sequence encoding the Gaussian luciferase having the sequence of SEQ ID NO: 1, the third nucleotide of each codon was diversified to introduce variations into mRNA codons. Nomenclature for International Union of Pure and Applied Chemistry (IUPAC) ambiguity codes, which is commonly used by those of ordinary skill in the art, was used to design and describe in detail variant sequences introduced into a sequence encoding the Gaussia luciferase gene, and was expressed as follows. N refers to adenine (A) or guanine (G) or cytosine (C) or thymine (T), R refers to adenine (A) or guanine (G), Y refers to cytosine (C) or thymine (T), H refers to adenine (A) or cytosine (C) or thymine (T), and D refers to adenine (A) or guanine (G) or T (thymine). In the mRNA sequences of Gaussian luciferase in which mutations were introduced into the third nucleotides of the codons, the ambiguity codes were used to express as follows: uuY for phenylalanine; uuR or cuN for leucine; auH for isoleucine; guN for valine; ucN or agY for serine; ccN for proline; acN for threonine; gcN for alanine; uaY for tyrosine; caY for histidine; caR for glutamine; aaY for asparagine; aaR for lysine; gaY for aspartic acid; gaR for glutamic acid; ugY for cysteine; cgN or agR for arginine; and ggN for glycine. In addition, methionine and tryptophan are expressed as atg and tgg and have only one codon. A template (variation-introduced GL gene template) including a coding sequence designed to contain the codon variations in SEQ ID NO: 1 was represented by SEQ ID NO: 2, and the sequences of SEQ ID NOs: 1 and 2 are shown in Table 1 (the sequence of the open reading frame was written in lowercase letters, and a portion where no mutation is introduced in the open reading frame was underlined.

**Table 1**

| **Classifi cation** | **Nucleotide sequence (5'-3')** | **SE Q ID NO:** |
|---|---|---|
| Wild-type IVT templat e | AGATCTAATACGACTCACTATAGGGAAATAAGAGAGAAAAGA AGAGTAAGAAGAAATATAAGAGAATTCGCCACC Atg gga gtg aaa gtt ctt ttt gcc ctt att tgt att gct gtg gcc gag gcc aaa cca act gaa aac aat gaa gat ttc aac att gta gct gta gct agc aac ttt gct aca acg gat ctc gat gct gac cgt ggt aaa ttg ccc gga aaa aaa tta cca ctt gag gta ctc aaa gaa atg gaa gcc aat gct agg aaa gct ggc tgc act agg gga tgt ctg ata tgc ctg tca cac atc aag tgt aca ccc aaa atg aag aag ttt atc cca gga aga tgc cac acc tat gaa gga gac aaa gaa agt gca cag gga gga ata gga gag gct att gtt gac att cct gaa att cct ggg ttt aag gat ttg gaa ccc atg gaa caa ttc att gca caa gtt gac cta tgt gta gac tgc aca act gga tgc ctc aaa ggt ctt gcc aat gtg caa tgt tct gat tta ctc aag aaa tgg ctg cca caa aga tgt gca act ttt gct agc aaa att caa ggc caa gtg gac aaa ata aag ggt gcc ggt ggt gat taa GGATCCGCTGCCTTCTGCGGGGCTTGCCTTCTGGCCATGCC CTTCTTCTCTCCCTTGCACCTGTACCTCTTGGTCTTTGAATAA AGCCTGAGTAGGAAGAATATTAAAAAAAAAAAAAAAAAAGCT T | 1 |
| Mutatio n-introduc ed IVT templat e | AGATCTAATACGACTCACTATAGGGAAATAAGAGAGAAAAGA AGAGTAAGAAGAAATATAAGAGAATTCGCCACC atg ggN gtN aaR gtN ctN ttY gcN ctN atH tgY atH gcN gtN gcN gaR gcN aaR ccN acN gaR aaY aaY gaR gaY ttY aaY atH gtN gcN gtN gcN tcN aac ttt gct aca acg gat ctN gaY gcN gaY cgN ggN aaR ctN ccN ggN aaR aaR ctN ccN ctN gaR gtN ctN aaR gaR atg gaR gcN aaY gcN cgN aaR gcN ggN tgY acN cgN ggN tgt ctg ata tgc ctg tca caY atH aaR tgY acN ccN aaR atg aaR aaR ttY atH ccN ggN cgN tgY caY acN taY gaR ggN gaY aaR gaR tcN gcN caR ggN ggN atH ggN gaR gcN att gtt gac att cct gaa atH ccN ggN ttY aaR gaY ctN gaR ccN atg gaR caR ttY atH gcN caR gtN gaY ctN tgY gtN gaY tgY acN acN ggN tgY ctN aaR ggN ctN gcN aaY gtg caa tgt tct gat tta ctN aaR aaR tgg ctN ccN caR cgN tgY gcN acN ttY gcN tcN aaR atH caR ggN caR gtN gaY aaR atH aaR ggN gcN ggN ggN gaY taa GGATCCGCTGCCTTCTGCGGGGCTTGCCTTCTGGCCATGCC CTTCTTCTCTCCCTTGCACCTGTACCTCTTGGTCTTTGAATAA AGCCTGAGTAGGAAGAATATTAAAAAAAAAAAAAAAAAAGCT T | 2 |

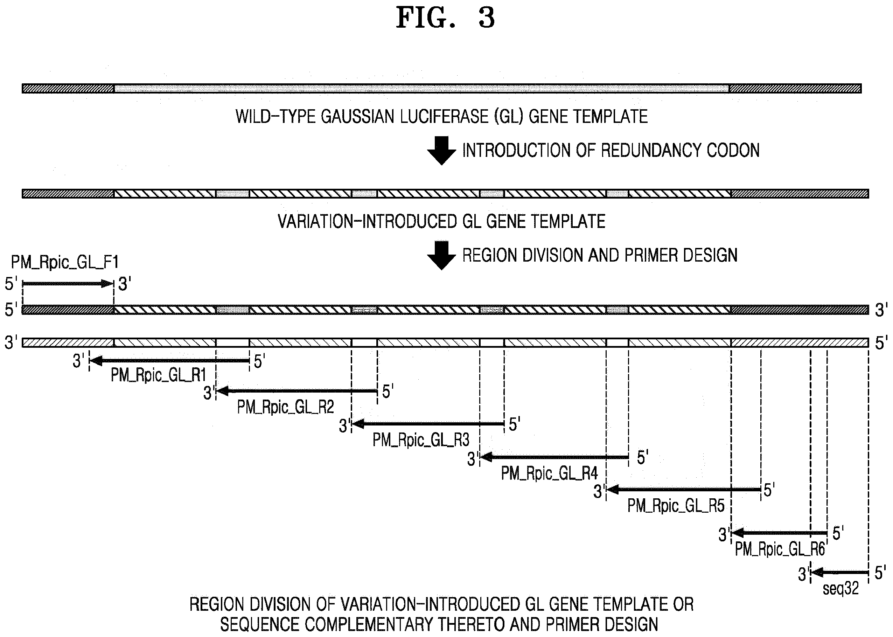

Next, to produce the template DNA sequence of SEQ ID NO: 2 on the basis of the sequence of SEQ ID NO: 1, first, the entire coding sequence of SEQ ID NO: 2, which includes about 760 base pairs, and a sequence complementary thereto were divided into a size of about 75 nts to about 135 nts, and each divided region was synthesized (FIG. 3). Information on the synthesized oligonucleotide sequences is shown in Table 2.

**Table 2**

| **Classificati on** | **Name** | **Nucleotide sequence (5'-3')** | **SE Q ID NO:** |
|---|---|---|---|
| First primer | PM_Rpic_G L_F1 | AGATCTAATA CGACTCACTA TAGGGAAATA AGAGAGAAAA GAAGAGTAAG AAGAAATATA AGAGAATTCG CCACC | 3 |
| Second primer | PM_Rpic_G L_R1 | ATC CGT TGT AGC AAA GTT nga ngc nac ngc nac dat rtt raa rtc ytc rtt rtt ytc ngt ngg ytt ngc ytc ngc nac ngc dat rca dat nag ngc raa nag nac ytt nac ncc cat GGT GGC GAA TTC TCT TAT | 4 |
| Third primer | PM_Rpic_G L_R2 | TGA CAG GCA TAT CAG ACA ncc ncg ngt rca ncc ngc ytt ncg ngc rtt ngc ytc cat ytc ytt nag nac ytc nag ngg nag ytt ytt ncc ngg nag ytt ncc ncg rtc ngc rtc nag ATC CGT TGT AGC AAA GTT | 5 |
| Fourth primer | PM_Rpic_G L_R3 | TTC AGG AAT GTC AAC AAT ngc ytc ncc dat ncc ncc ytg ngc nga ytc ytt rtc ncc ytc rta ngt rtg rca ncg ncc ngg dat raa ytt ytt cat ytt ngg ngt rca ytt dat rtg TGA CAG GCA TAT CAG ACA | 6 |
| Fifth primer | PM_Rpic_G L_R4 | TAA ATC AGA ACA TTG CAC rtt ngc nag ncc ytt nag rca ncc ngt ngt rca rtc nac rca nag rtc nac ytg ngc dat raa ytg ytc cat ngg ytc nag rtc ytt raa ncc ngg dat TTC AGG AAT GTC AAC AAT | 7 |
| Sixth primer | PM_Rpic_G L_R5 | GCA GAA GGC AGC GGA TCC tta rtc ncc ncc ngc ncc ytt dat ytt rtc nac ytg ncc ytg dat ytt nga ngc raa ngt ngc rca ncg ytg ngg nag cca ytt ytt nag TAA ATC AGA ACA TTG CAC | 8 |
| Seventh primer | PM_Rpic_G L_R6 | CTCAGGCTTT ATTCAAAGAC CAAGAGGTAC AGGTGCAAGG GAGAGAAGAA GGGCATGGCC AGAAGGCAAG CCCCGCAGAA | 9 |
| | | GGCAGCGGAT CC | |
| Eighth primer | Seq32 | TTTTTTTTTT TTTTTTTTTT TTTTTTTTTT TTTTTTTTTT TTTTTTTTTT TTTTT TTTTT TTTTTAATAT TCTTCCTACT CAGGCTTTAT TCAAAGACC | 10 |
| - | Seq30 | AGATCTAATA CGACTCACTA TAGGGAAATA AGA | 11 |

Next, sequential PCR was performed by using the synthesized oligonucleotide sequences as primers (FIG. 4). First, PCR was performed by using PM_Rpic_GL_F1 (SEQ ID NO: 3) and PM_Rpic_GL_R1 (SEQ ID NO: 4) as primers. In this case, the 3' end portion of PM_Rpic_GL_F1 and the 3' end portion of PM_Rpic_GL_R1 complementarily bind to each other to perform PCR, and a result of the reaction, a sequence corresponding to a partial region of SEQ ID NO: 2 is produced as a product (SEQ ID NO: 12).

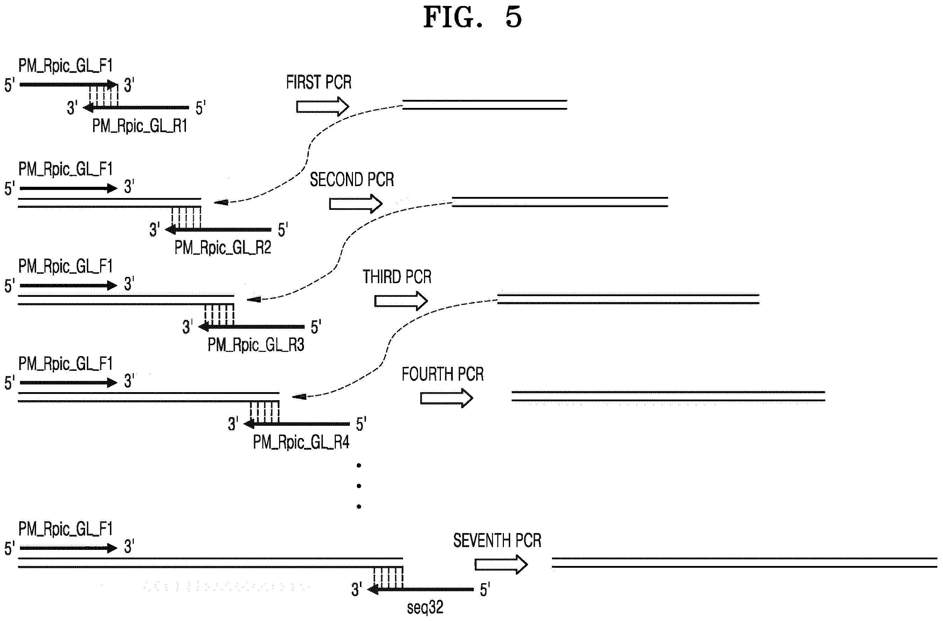

Subsequently, second PCR was performed by using the sequence of SEQ ID NO: 12 as a template and using PM_Rpic_GL_F1 (SEQ ID NO: 3) and PM_Rpic_GL_R2 (SEQ ID NO: 5) as primers, in which the 3' end portion of SEQ ID NO: 12 and the 3' end portion of PM_Rpic_GL_R2 complementarily bind to each other, and as a result, a product of SEQ ID NO: 13 is produced. As such, by performing sequential PCR by using a product of the previous PCR as a template, a sequence of SEQ ID NO: 2 can be finally produced (FIG. 5). Sequence information of the templates, primers, and products of the sequential PCR is shown in Table 3.

**Table 3**

| **PCR order** | **Template** | **Primer-forward** | **Primer-reverse** | **Reaction product** |
|---|---|---|---|---|
| First | - | PM_Rpic_GL_F1 (SEQ ID NO: 3) | PM_Rpic_GL_R1 (SEQ ID NO: 4) | SEQ ID NO: 12 |
| Second | SEQ ID NO: 12 | PM_Rpic_GL_F1 (SEQ ID NO: 3) | PM_Rpic_GL_R2 (SEQ ID NO: 5) | SEQ ID NO: 13 |
| Third | SEQ ID NO: 13 | PM_Rpic_GL_F1 (SEQ ID NO: 3) | PM_Rpic_GL_R3 (SEQ ID NO: 6) | SEQ ID NO: 14 |
| Fourth | SEQ ID NO: 14 | PM_Rpic_GL_F1 (SEQ ID NO: 3) | PM_Rpic_GL_R4 (SEQ ID NO: 7) | SEQ ID NO: 15 |
| Fifth | SEQ ID NO: 15 | PM_Rpic_GL_F1 (SEQ ID NO: 3) | PM_Rpic_GL_R5 (SEQ ID NO: 8) | SEQ ID NO: 16 |
| Sixth | SEQ ID NO: 16 | PM_Rpic_GL_F1 (SEQ ID NO: 3) | PM_Rpic_GL_R6 (SEQ ID NO: 9) | SEQ ID NO: 17 |
| Seventh | SEQ ID NO: 17 | PM_Rpic_GL_F1 (SEQ ID NO: 3) | Seq32 (SEQ ID NO: 10) | SEQ ID NO: 18 |

The final product of the sequential PCR is a library including various combinations of mutated codons. Specifically, the mutated codons introduced into SEQ ID NO: 18 as the final product correspond to a total of 160: 83 of N where four mutations are induced; 10 of H where three mutations are induced; and 31 of Y and 36 of R, where two mutations are induced, and thus, a library including sequences including about 8.14 × 10^74 (=(4^83) × (3^10) × (2^31) × (2^36)) of variant codons may be constructed. Thus, by identifying sequences that can interact with stabilized structures or stabilizing factors among the library sequences, an mRNA sequence with improved stability may be selected.

### Example 2: In vitro transcription (IVT) and purification steps for mutated mRNA production

To produce mRNA through *in vitro* transcription (IVT) from various variant sequences included in the library constructed according to Example 1, the following experiment was performed.

Specifically, to perform the *in vitro* transcription reaction, the HiScribe T7 ARCA mRNA kit with tailing from New England Biolabs, USA, which is a kit for mRNA production, was used. First, 1 µg of a library mixture including the mutant DNA produced according to Example 1, 10 µl of 2x ARCA/NTP Mix, and 2 µl of T7 RNA Polymerase Mix were mixed, and distilled water was added for the remainder, thereby finally obtaining 20 µl of a reaction mixture, and an IVT transcription reaction was allowed to occur at 37 °C for 30 minutes. When the reaction was completed, 2µl of DNase enzyme was added and treated at 37 °C for 15 minutes to remove all the residual DNA. After DNA removal was completed, to carry out a reaction to add the polyadenylic acid tail, 20 µl of the IVT reaction product was mixed with 5 µl Poly(A) polymerase reaction buffer and 5 µl of poly(A) polymerase, and distilled water was added for the remainder to thereby finally obtain 50 µl of a reaction mixture, and a reaction was allowed to occur at 37 °C for 30 minutes. The finally obtained IVT mRNA was purified by using an RNA purification kit.

### Example 3: Transfection of IVT mRNA into host cells

To transfect the IVT mRNA produced according to Example 2 into the human cell line HEK293T (ATCC, CRL-3216) and express the same, the following experiment was performed.

Specifically, first, to culture HEK293 cells, the cells were cultured in an MEM medium supplemented with 10% FBS (Fetus Bovine Serum: GIBCO, #16000-028) and 1 % ABAM (GIBCO BRL, #15240-O13) in a T75 flask for 3 days, followed by washing with 1 x PBS buffer and treatment with trypsin, and the cultured cells were separated from the bottom of the culture vessel. The separated cells were centrifuged to remove the supernatant, was redispersed in 1 x PBS buffer, and then redispersed in an electroporation solution at a concentration of about 5 x 10⁵ cells/ml, about 20 µg of IVT mRNA was added to 1 ml of the solution in which the cells were dispersed and mixed therewith, and transfection was carried out by using the Neon Transfection System from Thermo Fisher Scientific, USA. The conditions for performing electroporation were in accordance with the manufacturer's protocol. The transfected cells were redispersed in a 6-well microplate container and cultured in a 5% CO₂ cell incubator.

### Example 4: Isolation/purification of mRNA with excellent in vivo stability in transfected cells

To obtain an mRNA sequence with excellent intracellular stability from the transfected cells prepared according to Example 3 and purify the same, the following experiment was performed.

Specifically, after transfection using the electroporation method in Example 3, the cells were cultured for 72 hours to select an RNA sequence with improved intracellular stability, and then the cultured cells and the cell culture medium were harvested. Next, to confirm whether the sequence encoding Gaussian luciferase is transfected well and expresses the target protein, the expression level of Gaussian luciferase secreted into the harvested cell culture medium was measured, a GLuc assay solution was prepared by adding 50 µl of BioLux GLuc Substrate and 800 µl of BioLux GLuc Stabilizer to 5 ml of BioLux GLuc Assay Buffer per 100 samples, 10 µl of the culture medium recovered at each time period was added into a 96-well microplate, 50 µl/well of the GLuc assay solution was added, followed by culture for 35 seconds to 40 seconds, and the luminescence was measured with a luminometer.

Next, intracellular RNA was isolated and purified from cells cultured for 72 hours after the transfection by using a method well known to those of ordinary skill in the art.

### Example 5: Amplification of cDNA from purified mRNA by using RT-PCR method

To synthesize/amplify cDNA from mRNA obtained from the transfected cells of Example 4, the following experiment was performed.

Specifically, cDNA was first synthesized using RNA as a template through RT-PCR using reverse transcriptase, and the cDNA was amplified by general polymerase chain reaction (PCR). First, to perform RT-PCR, 100 ng of Random hexamer, 2 µl of 10x reverse transcriptase buffer, 10 mM DTT, 10 mM dNTP, and 250 units of reverse transcriptase were added to 2 µg of RNA purified in Example 4, and then the final volume was adjusted to 20 µl with distilled water. The reaction mixture was incubated at 70 °C for 10 minutes to modify RNA, a reverse transcription reaction was performed at 42 °C for 1 hour, and reverse transcriptase was inactivated again at 80 °C for 15 minutes. The cDNA product was subjected to polymerase chain reaction again using the primers of SEQ ID NOs: 10 and 11 to amplify, isolate and purify DNA fragments for IVT mRNA production. Meanwhile, the DNA fragments include various mutant sequences, and thus may be considered a secondary library. The steps of Examples 3 to 5 may be repeated several times to construct a tertiary library, a quaternary library, and the like, and as the number of repetitions increases, a library consisting of RNA sequences with improved intracellular stability may be obtained.

### Example 6: IVT mRNA production from amplified cDNA and cell transfection

IVT mRNA was produced by the method of Example 2 by using the DNA fragments isolated/purified in Example 5, and transfected again into HEK293 cells. At this time, the same amount of IVT mRNA produced using the template sequence of SEQ ID NO: 1 with no introduced variation as a control was transfected. After 24, 48, and 72 hours, the expression levels of secretory luciferase in the transfected cells were compared and measured for each time period, through which it was confirmed that the mutated Gaussian luciferase mRNA coding sequences obtained through the examples exhibited improved stability compared to wild-type luciferase mRNA with no introduced mutation. It was also confirmed that the protein expression level significantly increased along with an increase in Gaussian luciferase protein translation efficiency. RNA was isolated and purified from cells with significantly increased stability and expression levels.

### Example 7: Cloning of cDNA synthesized from IVT mRNA and IVT mRNA reproduction and stability evaluation using same

Total RNA or mRNA obtained from the cells with significantly increased stability and expression levels confirmed in Example 6 was subjected to RT-PCR using the primers of SEQ ID NOs: 10 and 11 to amplify cDNA. The cDNA amplified in this step contains a mixture of cDNAs reverse-transcribed from various types of stabilized mRNA, so it may be considered a more purified library. Therefore, to select a specific sequence of the stabilized mRNA among the various sequences, analyze the coding sequence, and test the degree of stabilization, there is a need to clone each corresponding cDNA sequence into a T vector system for *E*. *coli.* To this end, a T&A cloning vector available from Yeastern Biotech, Taiwan, which is well known to those of ordinary skill in the art, was used, plasmid vector DNA was isolated and purified from a clone into which each cDnA was inserted, and an *in vitro* transcription reaction was performed for each clone by using the above method to produce IVT mRNA. The selected and produced IVT mRNAs and control IVT mRNAs were transfected for each clone into HEK293T cells by using the above electroporation method, the culture media were collected over time, and the expression level of luciferase was analyzed to select clones exhibiting excellent luciferase expression levels compared to the controls.

### Example 8: Evaluation and determination of variant coding codons of which stability has been confirmed

The coding sequences of the clones selected as showing excellent expression levels above were confirmed by using a sequencing method well known to those skilled in the art. By using the sequencing results, a common coding sequence may be derived by comparing the coding sequences of various clones, or a novel meaningful structure may be analyzed through an RNA tertiary structure prediction program (http://rna.tbi.univie.ac.at/cgi-bin/RNAWebSuite/RNAfold.cgi). It is also possible to predict or discover proteins capable of binding to the RNA tertiary structure and regulate the stability of mRNA.

Specifically, tens of mRNA coding sequences obtained through a series of IVT mRNA reproduction and stability evaluation processes of Gaussian luciferase in Example 7 were selected, and among these, the coding sequences of eight clones was summarized in SEQ ID NOs: 19 to 26. As a result of comparing the similarity between the original wild-type coding sequence (SEQ ID NO: 1) and the sequence of each clone, the similarity of the coding sequences was analyzed to be within a range of 79.0 % to 82.6 % with no change in the amino acid sequence (Table 4).

**Table 4**

| **Clone** | **Code sequence similarity (%)** |
|---|---|
| RPIC_GL_3 | 81.2 |
| RPIC_GL_5 | 82.3 |
| RPIC_GL_7 | 82.4 |
| RPIC_GL_9 | 82.4 |
| RPIC_GL_15 | 82.6 |
| RPIC_GL_28 | 82.4 |
| RPIC_GL_32 | 79.0 |
| RPIC_GL_34 | 81.2 |

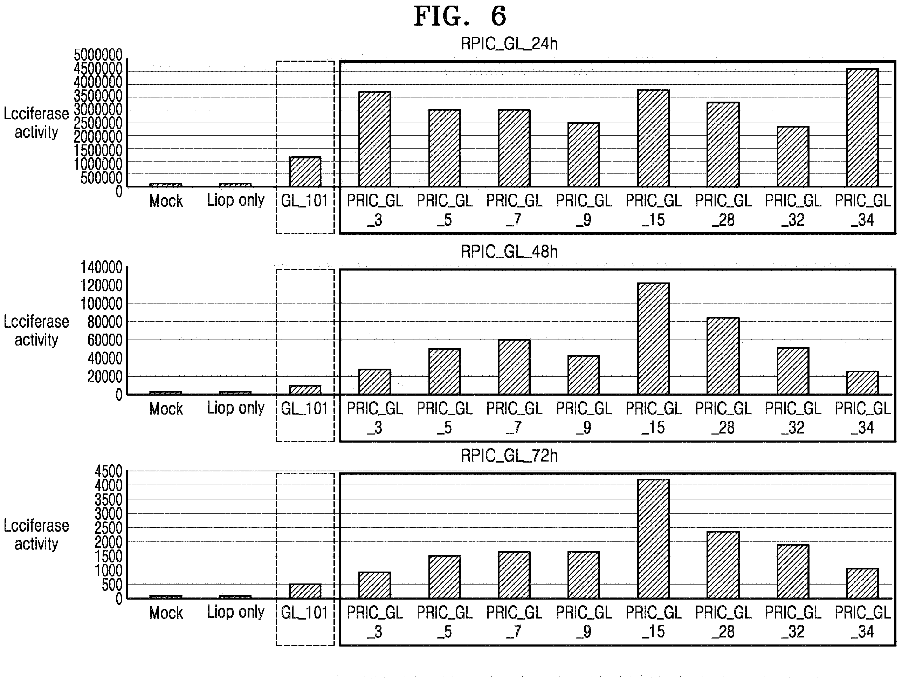

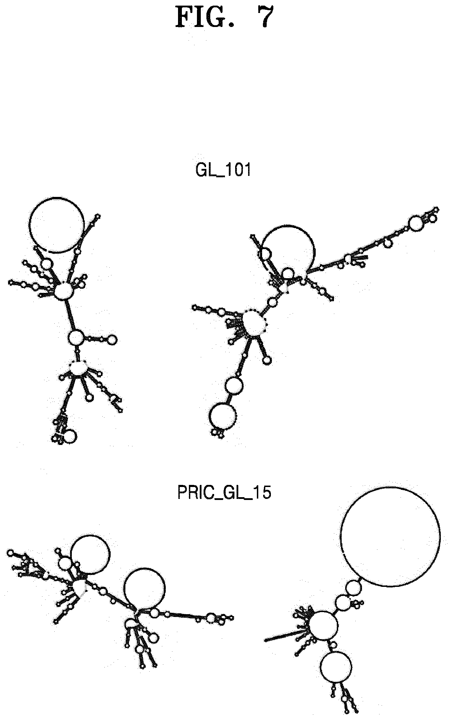

It is also confirmed that, among the selected clones, RPIC_GL_15 exhibits a very high expression level and stability compared to the controls, not only 24 hours but also 48 hours and 72 hours after the transfection (FIG. 6), through which it can be seen that, among various variant sequences, RPIC_GL_15 exhibits excellent intracellular stability. Next, as a result of analyzing the RNA coding sequence of RPIC_GL_15 by using the above-mentioned RNA structure-determining algorithm program, the RNA coding sequence showed a structure very different from the secondary structure of the original coding sequence (FIG. 7). On the basis of the above results, it can be seen that the series of processes of the present disclosure can select cDNA or mRNA that is structurally different in cells and has significantly improved stability, and can improve the production of a protein using the selected sequence.

### Example 9: Using method of screening for and producing mRNA with improved intracellular stability

To confirm whether the method of screening for mRNA of the present disclosure, which showed an excellent effect in the above examples, can be applied to target proteins other than Gaussian luciferase, the following experiment was performed.

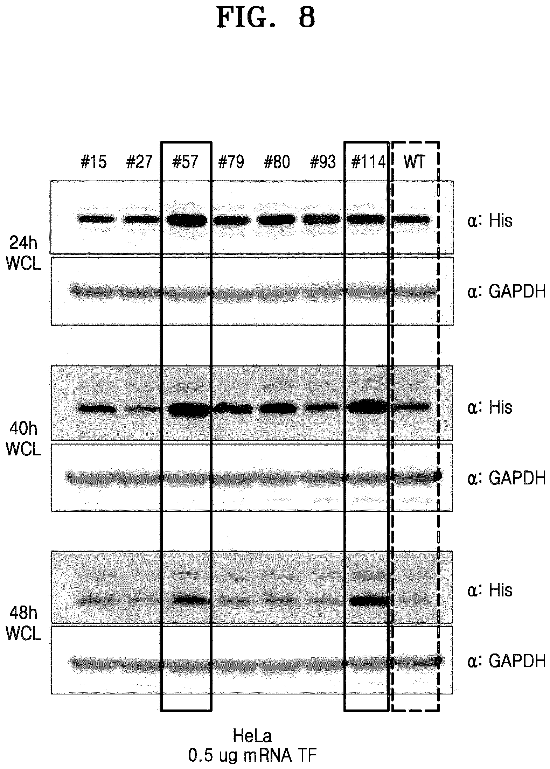

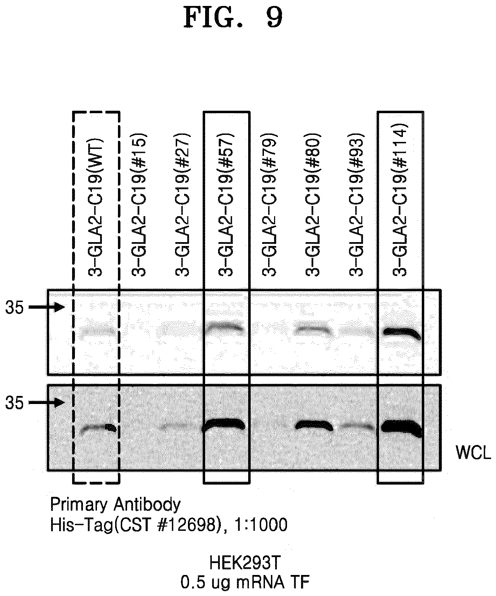

First, the selection of mRNA with improved intracellular stability encoding a Covid19 antigen protein was attempted by using the Covid19 antigen protein as the target protein. Specifically, mRNA with improved intracellular stability was effectively screened for through the method of screening for mRNA described in the above examples, using a template (SEQ ID NO: 27) including the coding sequence of the Covid19 antigen protein with no introduced mutation. As a result, it was confirmed that, among sequences in which variations have been introduced into nucleotide sequences encoding the target protein such that there is no change in the amino acid sequence of the target protein, the cases of #57 (SEQ ID NO: 30) and #114 (SEQ ID NO: 34) exhibited excellent intracellular stability compared to a wild-type sequence (WT), and the selected sequences showed the same result in both Hela cells and HEK293T cells (FIGS. 8 and 9).

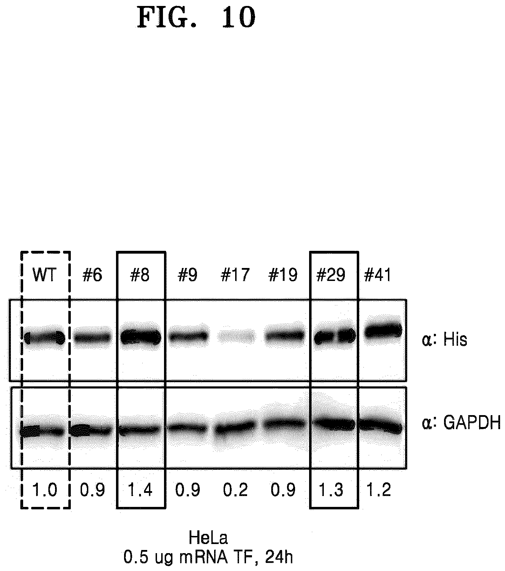

In addition, as a result of screening for mRNA with improved intracellular stability through the above-described method using, as the target protein, a recombinant protein including the Covid19 antigen protein and additional peptides, the same result was obtained, through which it was confirmed that, among sequences in which mutations had been introduced into nucleotide sequences encoding the target protein such that there is no change in the amino acid sequence of the target protein, the cases of #8 (SEQ ID NO: 36) and #29 (SEQ ID NO: 39) exhibited excellent intracellular stability compared to a wild-type sequence (WT) (FIG. 10).

On the basis of the above results, it can be seen that the method of screening for mRNA of the present disclosure can effectively screen for a sequence with improved intracellular stability regardless of the type and sequence of the target protein.

The aforementioned description of the present disclosure is for illustrative purposes, and it will be understood by those of ordinary skill in the art to which the present disclosure pertains that modifications can be easily made in other specific forms without changing the technical idea or essential characteristics of the present disclosure. Therefore, it should be understood that the examples described herein are considered in a descriptive sense only and not for purposes of limitation in all aspects.

## Claims

1. A method of screening for an mRNA sequence with improved intracellular stability, the method comprising:
(A) designing a template sequence comprising a sequence in which a variation has been introduced into a nucleotide sequence encoding a target protein such that there is no change in an amino acid sequence of the target protein;
(B) producing primers on the basis of the designed template sequence;
(C) constructing a library comprising various sequences into which variations have been introduced, by using sequences of the produced primers; and
(D) selecting a sequence with improved intracellular stability from the constructed library.

2. The method of claim 1, wherein the introduction of the variation in step (A) comprises introducing a redundancy codon encoding a same amino acid into all or part of the nucleotide sequence encoding the target protein.

3. The method of claim 1, wherein the introduction of the variation in step (A) comprises introducing N (A, G, C, or T), R (A or G), Y (C or T), or H (A, C, or T) into third nucleotides of codons in all or part of the nucleotide sequence encoding the target protein.

4. The method of claim 1, wherein the template sequence designed in step (A) further comprises at least one selected from the group consisting of a promoter, an untranslated region (UTR), a 5' cap, and a 3' polyadenylic acid sequence (poly-A).

5. The method of claim 1, wherein step (A) further comprises setting the designed template sequence as two or more divided regions,
wherein ends of each of the divided regions overlap each other.

6. The method of claim 5, wherein the overlapping portions of each of the divided regions have no introduced variations.

7. The method of claim 5, wherein the setting of the designed template sequence as two or more divided regions comprises dividing the template sequence into regions having a size of 10 bp to 1,000 bp, and regions having a size of 1 bp to 100 bp of the ends of each of the divided regions overlap each other.

8. The method of claim 1, wherein step (B) comprises synthesizing sequences of divided regions of the designed template sequence or sequences complementary thereto as primers.

9. The method of claim 8, wherein the synthesizing of the primers comprises synthesizing, as primers, complementary sequences in remaining regions excluding a first region from the 5' end.

10. The method of claim 1, wherein step (C) comprises performing sequential PCR by using primer sequences produced in step (B) and PCR products.

11. The method of claim 1, wherein step (D) comprises:
D-1) transfecting cells with a DNA sequence encoding the target protein included in the library or an RNA sequence produced therefrom;
D-2) obtaining an RNA sequence encoding the target protein from the transfected cells; and
D-3) constructing a library by synthesizing DNA encoding the target protein from the obtained RNA sequence.

12. The method of claim 11, wherein, in step (D), steps D-1) to D-3) are repeatedly performed one or more times.

13. The method of claim 11, wherein step (D) further comprises:
D-4) cloning the DNA sequence encoding the target protein included in the library into a vector;
D-5) transfecting cells with the vector or RNA produced therefrom; and
D-6) obtaining RNA encoding the target protein from the transfected cells.

14. The method of claim 13, wherein step (D) further comprises D-7) determining sequences of the obtained RNA and DNA synthesized therefrom.

15. The method of claim 1, wherein the target protein or mRNA encoding the target protein is used as a cancer immunotherapeutic agent, a cancer therapeutic vaccine, an infectious disease vaccine, a protein replacement therapeutic agent, an allergy therapeutic agent, or an immune disease therapeutic agent.
